(19) Europäisches Patentamt
European Patent Office
Office européen des brevets

(11) Numéro de publication: **0 082 040**
**B1**

(12) # FASCICULE DE BREVET EUROPÉEN

(45) Date de publication du fascicule du brevet: **15.01.86**

(21) Numéro de dépôt: **82402172.9**

(22) Date de dépôt: **29.11.82**

(51) Int. Cl.⁴: **C 07 D 471/16,** A 61 K 31/55 // (C07D471/16, 223:00, 221:00, 209:00)

(54) **Dérivés de diaza-3,7a cyclohepta(j,k)fluorènes, leur préparation et leur application en thérapeutique.**

(30) Priorité: **03.12.81 FR 8122643**

(43) Date de publication de la demande: **22.06.83 Bulletin 83/25**

(45) Mention de la délivrance du brevet: **15.01.86 Bulletin 86/3**

(84) Etats contractants désignés: **AT BE CH DE FR GB IT LI LU NL SE**

(56) Documents cité: **FR-A-2 442 236**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Bertin, Jean, 55, rue de'Estienne d'Orves, F-92140 Clamart (FR)**
Inventeur: **Frost, Jonathan, 15, rue des Amandiers, F-92340 Cachan (FR)**

(74) Mandataire: **Ludwig, Jacques, SYNTHELABO Service Brevets 58, rue de la Glacière, F-75621 Paris Cedex 13 (FR)**

**EP 0 082 040 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne des dérivés de diaza-3,7a cyclohepta [j,k] fluorènes, leur préparation et leur application en thérapeutique.

Les composés de l'invention répondent à la formule (I)

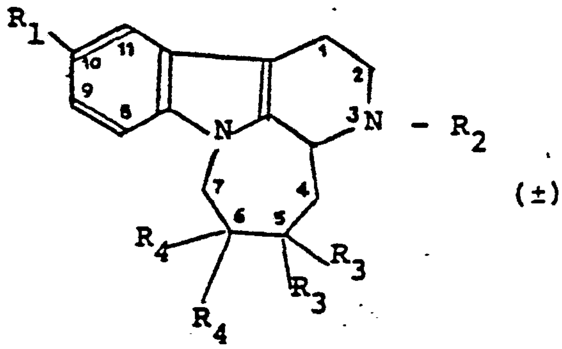

(I)

dans laquelle

$R_1$ représente un atome d'hydrogène ou de chlore ou un radical méthoxy,

$R_2$ représente un atome d'hydrogène, le radical méthyle ou benzyle, et

soit $R_3$ et $R_4$ représentent des atomes d'hydrogène, soit $R_3$ est $CH_3$ et $R_4$ est H, soit $R_3$ est H et $R_4$ est $CH_3$.

Les composés de l'invention existent sous la forme de racémates ou d'énantiomères, qui font partie de l'invention. Les sels d'addition à des acides pharmacologiquement acceptables des composés (I) font partie également de l'invention.

Les composés préférés de l'invention sont ceux dans la formule (I) desquels $R_1$, $R_2$, $R_3$ et $R_4$ représentent chacun un atome d'hydrogène.

Selon l'invention, on peut préparer les composés de l'invention selon le schéma réactionnel suivant:

**Schéma**

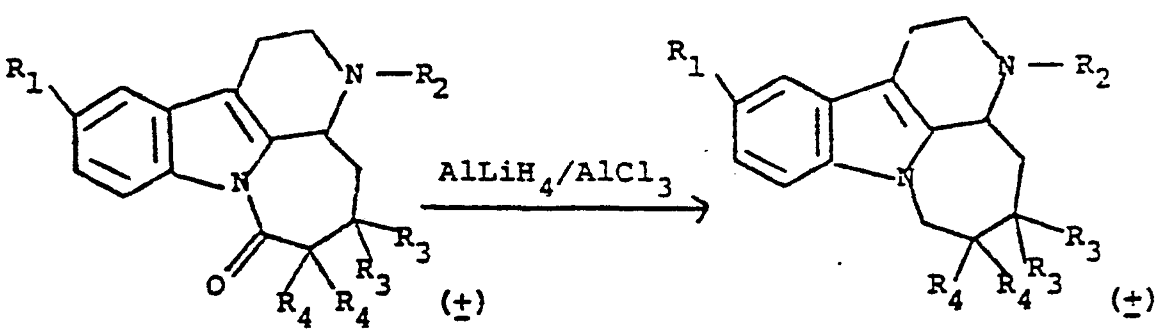


(II) (I)

On reduit le composé de départ (II) à l'aide d'un mélange de $AlLiH_4/AlCl_3$ pour obtenir le composé (I) Si on desire obtenir un composé (I) dans lequel $R_2$ est H, on peut aussi débenzyler par une hydrogénation catalytique 10 composé (I) correspondant dans lequel $R_2$ est $C_6H_5CH_2$.

Les composés de depart dans lesquels $R_1$, $R_3$ et $R_4$ sont des atomes d'hydrogène sont decrits dans la demande de brevet français n° 2 442 236. Les autres composes de départ sont decrits dans la demande de brevet français déposée par la Demanderesse publiée sous le n° 25 176878.

Les exemples ci-après illustrent la préparation des composés de l'invention.

Les analyses et les spectres IR et RMN ont confirme la structure des composés.

**Exemple 1**

Benzyl-3 octahydro-1,2,3,3a,4,5,6,7 diaza-3,7a cyclohepta [j,k] fluorène.

Dans 10 ml de tétrahydrofuranne, on introduit 0,285 g de $AlCl_3$, puis on ajoute 0,114 g d'$AlLiH_4$ et ensuite, par petites quantités, 0,33 g de benzyl-3 oxo-7 octahydro-1,2,3, 3a,4,5,8,7 diaza-3,7a cyclohepta [j,k] fluorene. On agite à la température ambiante pendant une heure. On ajoute 1,5 ml de soude concentrée, puis de l'acétate d'éthyle. On filtre, décante, lave à l'eau, sèche sur $Na_2SO_4$ puis évapore à sec.

On fait recristalliser le produit obtenu dans de l'éther isopropylique.

F = 104-105°C

**Exemple 2**

Octahydro-1,2,3,3a,4,5,8,7 diaza-3,7a cyclohepta [j,k] fluorène et son chlorhydrate.

Dans un appareil de Parr, on hydrogène sous une pression de 0,35 MPa, à la température ambiante, 1,5 g du composé obtenu dans l'exemple 1 en solution dans 50 ml de méthanol en présence du catalyseur Pd/C à 10% et d'acide chlorhydrique. Après filtration du catalyseur, on évapore le mélange réactionnel.

On filtre le chlorhydrate forme et le fait recristalliser dans du méthanol.

F = 294-296°C (déc.)

**Exemple 3**

Chloro-10 octahydro-1,2,3,3a,4,5,6,7 diaza-3,7a cyclohepta [j,k] fluorène.

A 25 ml de tétrahydrofuranne contenus dans un ballon tricol de 100 ml, sous atmosphère d'azote, on ajoute 0,586 g de chlorure d'aluminium, et on agite pendant 30 mn jusqu'à la dissolution. On ajoute ensuite 0,250 g d'hydrure d'aluminium et de lithium, en continuant d'agiter pendant 30 minutes. On introduit ensuite à la spatule 0,8 g de chloro-10 oxo-7 octahydro-1,2,3,3a,4,5,8,7 diaza-3,7a cyclohepta [j,k] fluorène, et on laisse sous agitation à température ambiante pendant deux heures, puis au repos pendant une nuit. Ensuite on introduit avec précaution 4 ml de soude concentrée, puis 30 ml d'eau, et l'on extrait par l'acétate d'éthyle. La phase organique est lavée à l'eau, séchée sur sulfate de magnésium puis évaporée à sec. On passe l'huile ainsi obtenue sur une colonne de silice avec un mélange de chloroforme et de méthanol 90/10 comme éluant. L'huile ainsi recueillie cristallise dans le pentane. Les cristaux fondent à 94-6°C.

Le tableau ci-après illustre d'autres composés conformes à l'invention, obtenus par des méthodes analogues.

**Tableau**

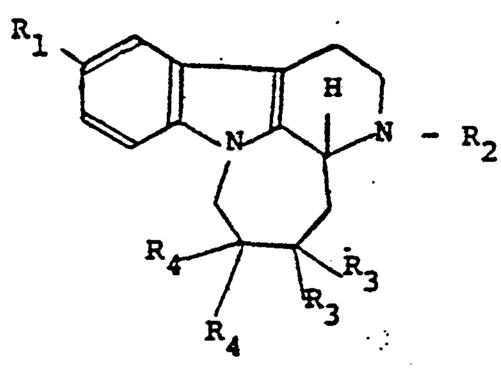

(I)

| Composé | $R_1$ | $R_2$ | $R_3$ | $R_4$ | Forme | F(°C) |
|---|---|---|---|---|---|---|
| 1 | H | $C_6H_5CH_2$ | H | H | base<br>HC1 | 104-5<br>218-20 |
| 2 | H | H | H | H | HC1 | 294-6 (d) |
| 3 | C1 | H | H | H | base | 94-6 |
| 4 | C1 | $C_6H_5CH_2$ | H | H | HC1 | 256-8 |
| 5 | H | $CH_3$ | $Ch_3$ | H | HCCOOH<br>‖<br>HCCOOH | 175 |
| 6 | H | $C_6H_5CH_2$ | $CH_3$ | H | HC1 | 195 |
| 7 | H | $CH_3$ | H | $CH_3$ | HCCOOH<br>‖<br>HCCOOH | 198-9 |
| 8 | H | H | $CH_3$ | H | HCCOOH<br>‖<br>HCCOOH | 180-1 |
| 9 | H | $C_6H_5CH_2$ | H | $CH_3$ | ‖<br>HCCOOH | 196-8 |
| 10 | H | H | H | $CH_3$ | HCCOOH<br>‖<br>HCCOOH | 186-7 |
| 11 | $CH_3O$ | $C_6H_5CH_2$ | H | H | HCCOOH<br>‖<br>HCCOOH | 168-70 |
| 12 | $CH_3O$ | H | H | H | HC1 | 294-6 |

d): fusion avec décomposition

Les composés de l'invention ont été soumis à des essais pharmacologiques destinés à mettre en évidence leur intêret en thérapeutique.

**Toxcicité aiguë chez la souris**

Les composés sont administrés à doses croissantes aux animaux d'essai. La toxicité des composés est exprimée par la dose, en mg par kg de poids corporel, qui laisse vivants la moitié des animaux du lot correspondant à chaque essai. On constate ainsi que, par la voie intrapéritonéale, les $DL_{50}$ des composés s'échelonnent de 30 mg/kg à plus de 1000 mg/kg, tandis que par voie orale elles vont de 100 mg/kg à plus de 1000 mg/kg.

**Activité antianoxique**

Administrés par voie intrapéritonéale, les composés de l'invention prolongent la vie des souris placées en atmosphère appauvrie en oxygène (réalisation d'un vide partiel dans une enceinte close où la pression est amenée à 2,5.10 Pa (190 mm Hg) en 30 secondes à l'aide d'une pompe à vide).

L'activité des composés est exprimée par la $DA_{100}$, dose en mg/kg qui prolonge de 100 % la durée de vie des animaux traités, comparée à la durée de vie des animaux témoins.

Les $DA_{100}$ des composés de l'invention se situent entre 2,9 et 8,3 mg/kg.

**Test de l'ischemie globale chez la souris**

On mesure le temps de survie des animaux d'essai après leur avoir injecté, dans la veine caudale, 0,1 ml d'une solution saturée de chlorure de magnésium. L'arrêt cardiaque qui en résulte provoque l'ischémie cérébrale. Le "temps de survie" est l'intervalle de temps entre l'injection du chlorure de magnésium et le dernier mouvement inspiratoire de chaque souris, considéré comme l'indice ultime d'une fonction du système nerveux central.

On compare les temps de survie des animaux traités par les composés de l'invention, administrés par voie intrapéritonéale 10 minutes avant l'injection du chlorure de magnésium, et les temps de survie des animaux témoins auxquels on n'a administré que le véhicule des substances actives.

Les souris étant etudiées par groupe de 10, les moyennes des résultats de chaque groupe permettent de tracer une courbe, grâce à laquelle on détermine graphiquement la Dose Efficace 3, $DE_3$, exprimée en mg de substance active par kg de poids corporel, qui prolonge le temps de survie de 3 secondes.

Une augmentation de la durée de survie de 3 secondes est à la fois significative statistiquement et reproductible.

Les $DE_3$ des composés de l'invention vont de 3 à 30 mg/kg.

L'étude pharmacologique des composés de l'invention montre qu'ils possèdent une activité antianoxique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, pour le traitement des encéphalopathies métaboliques et pour le traitement des états dépressifs.

L'invention comprend par conséquent toutes compositions pharmaceutiques renfermant les composés de l'invention ou leurs sels comme principes actifs, en association avec tous excipients appropriés à leur administration en particulier par voie orale ou parentérale.

La posologie journalière peut aller de 1 à 100 mg par la voie parentérale, et de 5 à 500 mg par la voie orale, les unités de prise étant par exemple dosées de 1 à 100, mg de substance active.

**Revendications** pour les Etats contractants: BE, CH, DE, FR, GB, IT, LI, LU, NL, SE.

1. Composés sous forme de racémates ou d'énantiomères, qui répondent à la formule (I)

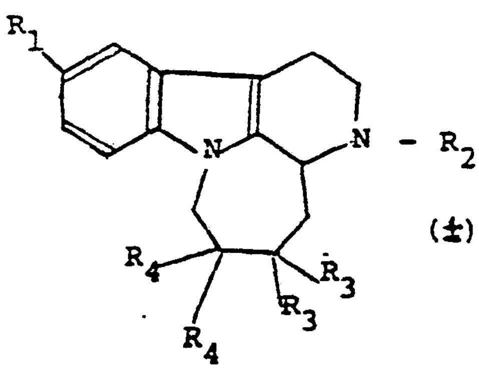

(I)

(±)

dans laquelle
$R_1$ représente un atome d'hydrogène ou de chlore ou un radical méthoxy,
$R_2$ représente un atome d'hydrogène, le radical méthyle ou benzyle, et
soit $R_3$ et $R_4$ représentent des atomes d'hydrogène, soit $R_3$ est $CH_3$ et $R_4$ est H, soit $R_3$ est H et $R_4$ est $CH_3$,
ainsi que leurs sels d'addition à des acides acceptables en pharmacologie.

2. Composés selon la revendication 1, caractérisés en ce que, dans la formule (I), $R_1$, $R_2$, $R_3$ et $R_4$ représentent chacun un atome d'hydrogène.

3. Procédé de préparation des composés de formule I selon la revendication 1, caractérisé en ce qu'on reduit un composé de formule (II)

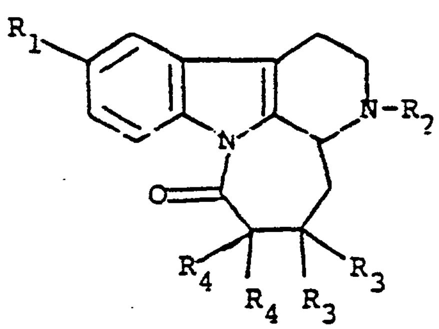

(II)

dans laquelle $R_1$, $R_2$, $R_3$ et $R_4$ sont tels que définis à la revendication 1, à l'aide d'un mélange d'hydrure d'aluminium et de lithium et de chlorure d'aluminium.

4. Procédé selon la revendication 3 pour la préparation de composés de formule (I) dans laquelle $R_2$ représente un atome d'hydrogène, procédé caractérisé en ce qu'on fait suivre la réduction d'un composé de formule (II), dans laquelle $R_2$ représente un groupe benzyle, par la débenzylation du composé obtenu, répondant à la formule

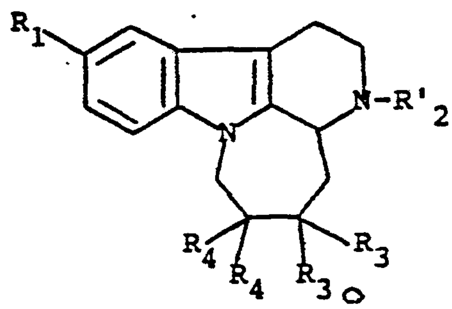

dans laquelle $R'_2$ représente un groupe benzyle, grâce à une hydrogénation catalytique.

5. Médicament, caractérisé en ce qu'il est constitué par un composé selon l'une des revendications 1 et 2.

6. Composition pharmaceutique, caractérisée en ce qu'elle contient un composé selon l'une des revendications 1 et 2, associé à un excipient acceptable en pharmacologie.

**Revendications** pour l'Etat contractant: AT

1. Procédé de préparation de composés, sous forme de racémates ou d'énantiomères, qui répondent à la formule (I)

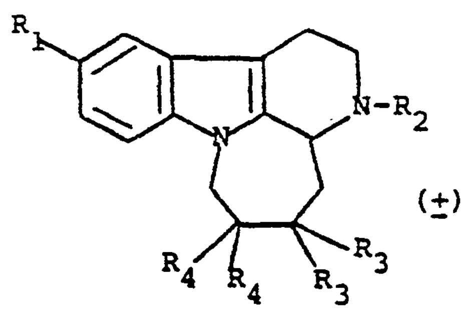

dans laquelle

$R_1$ représente un atome d'hydrogène ou de chlore ou un radical méthoxy,

$R_2$ représente un atome d'hydrogène, le radical méthyle ou benzyle, et

soit $R_3$ et $R_4$ représentent des atomes d'hydrogène, soit $R_3$ est $CH_3$ et $R_4$ est H, soit $R_3$ est H et $R_4$ est $CH_3$, ainsi que de leurs sels d'addition à des acides acceptables en pharmacologie,

procédé caractérisé en ce qu'on réduit un composé de formule (II)

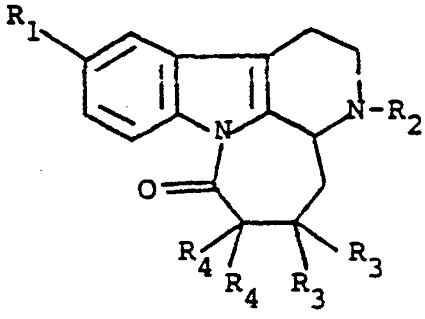

(II)

dans laquelle $R_1$, $R_2$, $R_3$ et $R_4$ sont tels que définis ci-dessus, à l'aide d'un mélange d'hydrure d'aliminium et de lithium et de chlorure d'aluminium.

2. Procédé selon la revendication 1 pour la préparation de composés de formule (I) dans laquelle $R_2$ représente un atome d'hydrogène, procédé caractérisé en ce qu'on fait suivre la réduction d'un composés de formula (II), dans laquelle $R_2$ représente un groupe benzyle, par la débenzylation du composé obtenu, répondant à la formule

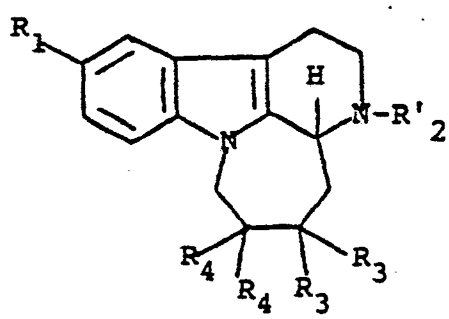

dans laquelle $R'_2$ représente un groupe benzyle, grâce à une hydrogénation catalytique.

**Claims** for the contracting states: BE, CH, DE, FR, GB, IT, LI, LU, NL, SE.

1. Compounds, in form of racemates or enantiomers, responding to formula (I)

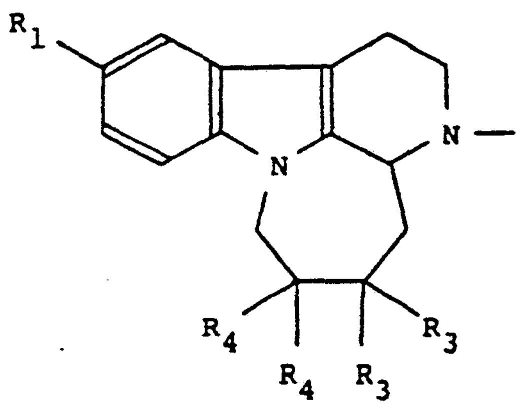

(I)

(±)

wherein

$R_1$ represents a hydrogen or chlorine atom or a methoxy radical,

$R_2$ represents a hydrogen atom or the methyl or benzyl radical, and

either $R_3$ and $R_4$ represent hydrogen atoms, or $R_3$ is $CH_3$ and $R_4$ is H, or $R_3$ is H and $R_4$ is $CH_3$,

and their addition salts with pharmacologically acceptable acids.

2. Compounds according to claim 1, characterized in that in formula (I), $R_1$, $R_2$, $R_3$ and $R_4$ each represent a hydrogen atom.

3. A process for the preparation of the compounds of formula I according to claim 1, characterized in that a compound of formula (II)

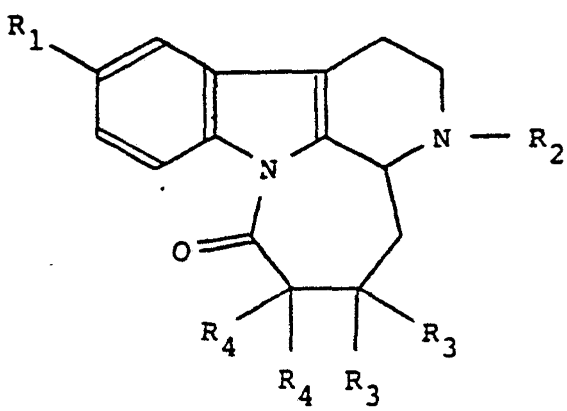

(II)

wherein $R_1$, $R_2$, $R_3$ and $R_4$ are as specified in claim 1, is reduced by means of a mixture of aluminium and lithium hydride and aluminium chloride.

4. A process according to claim 3 for the preparation of compounds of formula (I) wherein $R_2$ represents a hydrogen atom, process characterized in that the reduction of a compound of formula (II), wherein $R_2$ represents a benzyl group, is followed by the debenzylation of the compound obtained, responding to the formula

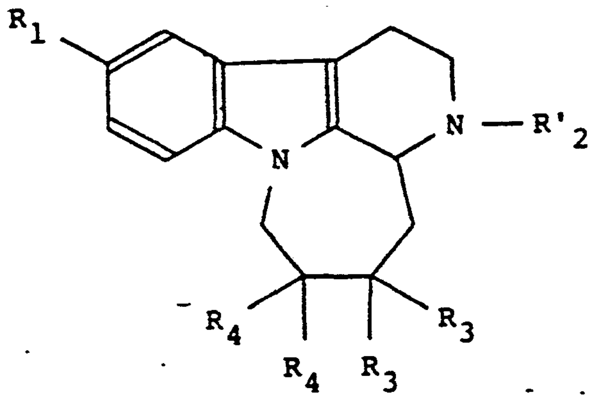

wherein $R'_2$ represents a benzyl group, by means of a catalytic hydrogenation.

5. A medicament, characterized in that it consists of a compound according to anyone of claims 1 and 2.

6. A pharmaceutical composition, characterized in that it contains a compound according to anyone of claims 1 and 2, in association with a pharmacologically acceptable carrier.

**Claims** for the contracting state: AT.

1. A process for the preparation of compounds, in form of racemates or enantiomers, responding to formula (I)

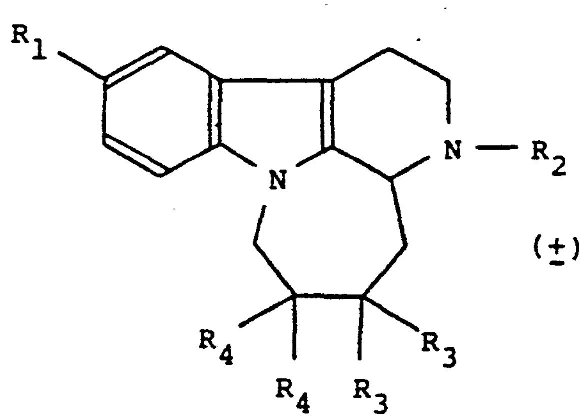

(I)

(±)

wherein
$R_1$ represents a hydrogen or chlorine atom or a methoxy radical,
$R_2$ represents a hydrogen atom or the methyl or benzyl radical, and
either $R_3$ and $R_4$ represent hydrogen atoms, or $R_3$ is $CH_3$ and $R_4$ is H, or $R_3$ is H and $R_4$ is $CH_3$,
and of their addition salts with pharmacologically acceptable acids,
characterized in that a compound of formula (II)

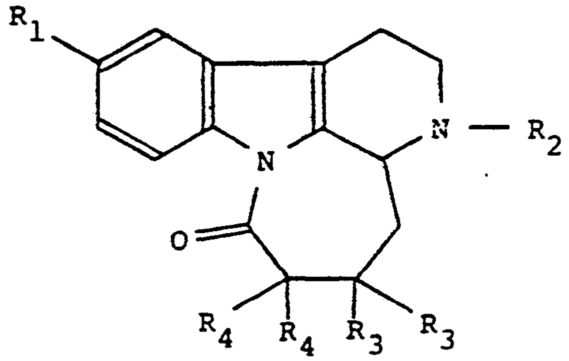

(II)

wherein $R_1$, $R_2$, $R_3$, and $R_4$ are as specified above, is reduced bv means of a mixture of aluminium and lithium hydride and aluminium chloride.

2. A process according to claim 1 for the preparation of compounds of formula (I) wherein $R_2$ represents a hydrogen atom, process characterized in that the reduction of a compound of formula (II), wherein $R_2$ represents a benzyl group, is followed by the debenzylation of the compound obtained, responding to the formula

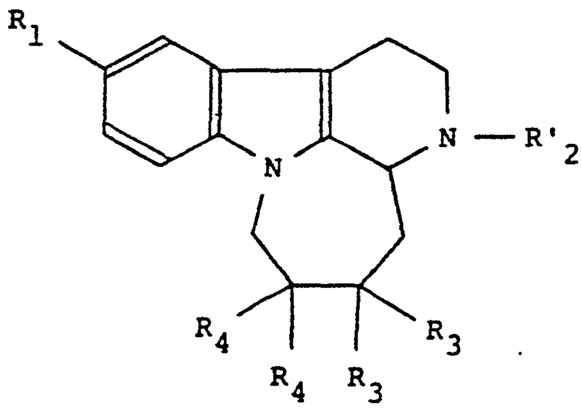

wherein $R'_2$ represents a benzyl group, by means of a catalytic hydrogenation.

**Patentansprüche** für die Vertragsstaaten: BE, CH, DE, FR, GB, IT, LI, LU, NL, SE.

1. Verbindungen in Form ihrer Racemate oder Enantiomeren, die der Formel

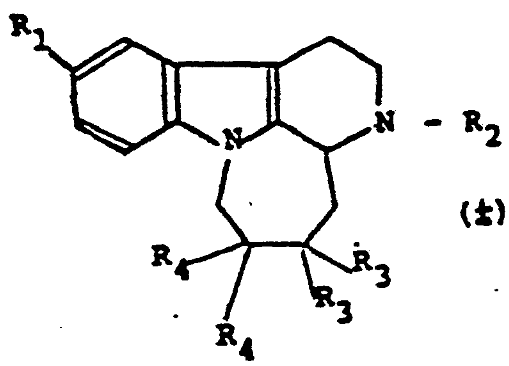

(I)

(±)

entsprechen, in welcher $R_1$ ein Wasserstoff- oder Chloratom oder einen Methoxyrest, $R_2$ ein Wasserstoffatom, den Methyloder Benzylrest bedeutet und $R_3$ und $R_4$ entweder Wasserstoffatome oder $R_3$ Methyl und $R_4$ Wasserstoff oder $R_3$ Wasserstoff und $R_4$ Methyl bedeuten, sowie ihre pharmakologisch verwendbaren Säureadditionssalze.

2. Verbindungen nach Anspruch 1
dadurch gekennzeichnet,
daß in der Formel (I) $R_1$, $R_2$, $R_3$ und $R_4$ jeweils ein Wasserstoffatom bedeuten.

3. Verfahren zur Herstellung von Verbindungen der Formel I nach Anspruch 1,
dadurch gekennzeichnet,
daß man eine Verbindung der Formel (II)

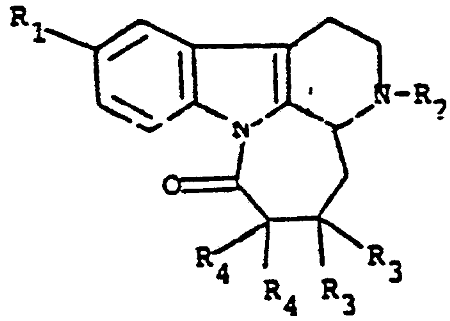

(II)

in welcher $R_1$, $R_2$, $R_3$ und $R_4$ die im Anspruch 1 genannte Bedeutung haben, mit Hilfe einer Mischung aus Aluminium- und Lithiumhydrid und Aluminiumchlorid reduziert.

4. Verfahren nach Anspruch 3 zur Herstellung von Verbindungen der Formel (I), in welcher $R_2$ für ein Wasserstoffatom steht, dadurch gekennzeichnet,

daß man an die Reduktion einer Verbindung der Formel (II), in welcher $R_2$ für eine Benzylgruppe steht, die Debenzylierung der erhaltenen Verbindungen der Formel

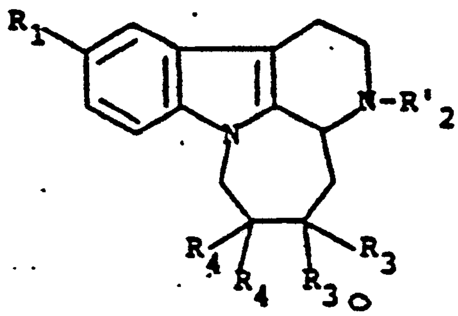

in welcher $R'_2$ eine Benzylgruppe bedeutet, mit Hilfe einer katalytischen Hydrierung anschließen läßt.

5.Heilmittel,
dadurch gekennzeichnet,
daß es aus einer Verbindung nach einem der Ansprüche 1 und 2 besteht.

6.Pharmazeutische Zusammensetzung,
dadurch gekennzeichnet,
daß sie eine Verbindung nach einem der Ansprüche 1 und 2 gemeinsam mit einem in der Pharmakologie verwendbaren Trägerstoff enthält.

**Patentansprüche** für den Vertragsstaat: AT

1. Verfahren zur Herstellung von Verbindungen der Formel (I)

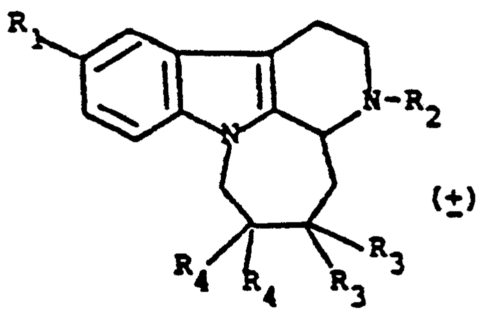

(±)

in welcher $R_1$ ein Wasserstoff- oder Chloratom oder einen Methoxyrest, $R_2$ ein Wasserstoffatom, den Methyl oder Benzyl rest bedeutet und $R_3$ und $R_4$ Wasserstoffatome oder $R_3$ Methyl und $R_4$ Wasserstoff oder $R_3$ Wasserstoff und $R_4$ Mehtyl bedeuten, in Form ihrer Racemate oder Enantiomeren sowie deren pharmakologisch verwendbaren Säureadditionssalzen

dadurch gekennzeichnet, daß man eine Verbindung der Formel (II)

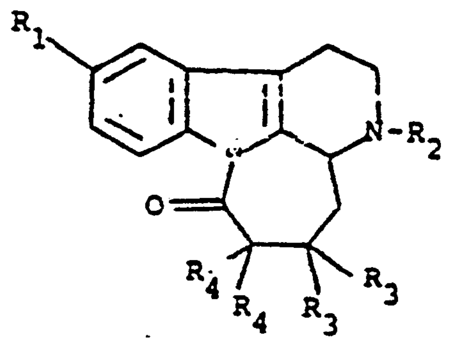

(II)

in welcher $R_1$, $R_2$, $R_3$ und $R_4$ die oben angegebene Bedeutung haben, mit Hilfe einer Mischung aus Aluminium- und Lithiumhydrid und Aluminiumchlorid reduziert.

2. Verfahren nach Anspruch 1 zur Herstellung von Verbindungen der Formel (I), in welcher $R_2$ für ein Wasserstoffatom steht, dadurch gekennzeichnet, daß man an die Reduktion einer Verbindung der Formel (II), in welcher $R_2$ für eine Benzylgruppe steht, die Debenzylierung der erhaltenen Verbindung

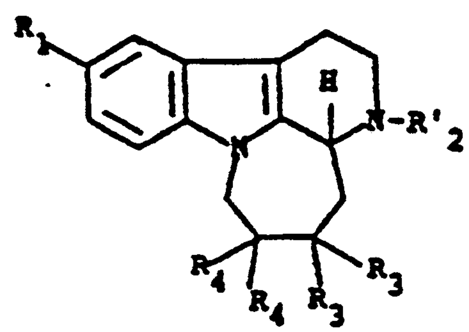

in welcher $R'_2$ eine Benzylgruppe bedeutet, mit Hilfe katalytischen Hydrierung anschließen läßt.